# EUROPÄISCHE PATENTSCHRIFT

(11) **EP 1 013 279 B1**
(45) Veröffentlichungstag und Bekanntmachung des Hinweises auf die Patenterteilung: **16.08.2001**
(21) Anmeldenummer: 99103424.0
(22) Anmeldetag: 23.02.1999
(51) Int. Cl.: A61K 35/78, A61K 7/48, A61K 47/46, C11D 7/44

(54) **Verfahren zur Herstellung einer mit Inhalts- und Wirkstoffen von Pflanzen angereicherten Flüssigkeit unter Verwendung eines Träger- und Speichermaterials für Pflanzen und deren Wirkstoffe; Herstellung von Flüssigkeiten daraus und Verwendung als kosmetisches, therapeutisches oder Reinigungsmittel**
Method for producing liquids enriched with ingredients and active agents from plants using a carrier and storage material for plants and their active agents, production of liquids from this material and use of said liquids as therapeutic, cosmetic or cleansing agents
Procédé de préparation de fluides enrichis en composés et agents actifs provenant de plantes utilisant un support et un matériau de stockage pour plantes et leurs agents actifs; préparation de fluides à partir de ceux-ci et utilisation comme agents cosmétiques, thérapeutiques ou de nettoyage

(30) Priorität: 24.02.1998 DE 19807731
(43) Veröffentlichungstag der Anmeldung: 28.06.2000
(73) Patentinhaber: Meilke, Gerd Rudolf, 06242 Krumpa (DE)
(72) Erfinder: Meilke, Gerd Rudolf, 06242 Krumpa (DE)
(74) Vertreter: Schulz, Manfred, Dipl.-Ing.

(56) Entgegenhaltungen:
- DE-A- 19 711 809
- DATABASE WPI Section Ch, Week 9244 Derwent Publications Ltd., London, GB; Class A97, AN 92-361469 XP002107622 & JP 04 262947 A (TAIHO KOGYO CO LTD), 18. September 1992 (1992-09-18)

## Beschreibung

Die Erfindung betrifft ein Verfahren zur Herstellung einer mit Inhalts- und Wirkstoffen von Pflanzen angereicherten Flüssigkeit unter Verwendung eines Träger- und Speichermaterials für Pflanzen und deren Wirkstoffe, für das Gummipulver aus Altreifen, Förderbändern und vergleichbarem Grundmaterial in reiner Form oder als Gemisch mit textilen Fasern als Träger- und Speichermittel verwendet wird, dadurch gekennzeichnet, daß das Altreifenmaterial mit der gesamten Pflanze vermischt wird, das Trägermaterial diese aufnimmt, das entstandene Gemisch getrocknet, gepreßt oder in Formen getrocknet wird, so daß es lange Zeit gelagert werden kann, ohne daß Wirkstoffverluste auftreten oder das Material verdirbt; für die Verwendung des Materials als kosmetisches Mittel, bzw. der Anwendung in Theraphie, Freizeit, Haushalt und Sport genügt die Ansetzung des Trägermaterials mit Flüssigkeit je nach Verwendungszweck in einem definierten Mischungsverhältnis , einer entsprechenden Temperatur und in einer bestimmten Zeiteinheit.

Bekannt sind Verfahren zur Herstellung von Pflanzenextrakten, bei denen versucht wird, die Wirk- und Inhaltsstoffe durch Auspressen oder Ausschleudern der Pflanzen, Ansetzen mittels Alkohol zu gewinnen und in flüssiger Form zu speichern. Bekannt ist auch die Methode des Trocknens von Pflanzen, wie es bei Heilkräutern und Tee üblich ist. Diese Verfahren besitzen den Nachteil, daß nur ein Teil der Wirkstoffe gebunden wird.

Bekannt sind auch chemische Verfahren, bei welchen die Wirkstoffe synthetisch gewonnen werden. Dabei wird jedoch immer nur der gewünschte Hauptwirkstoff hergestellt, ein Mix, wie er in der Natur vorkommt, ist nicht möglich bzw. zu aufwendig.

Weiter sind Verfahren zur Herstellung von Pflanzenextrakten durch Ausdampfen bekannt. So werden beispielsweise aus 6 Tonnen Teebaumlaub 15 Liter Teebaumöl gewonnen. Die Nachteile dieses Verfahrens sind ein sehr hoher Energieaufwand zur Verdampfung und der hohe Anteil an Restabfällen.

Bekannt ist auch ein Filter und Trägermaterial nach der DE-A-197 11809. Hierbei war die Verwendung des Trägermaterials als Speicher für medizinische und oder therapeutische Mittel und deren Verwendung in gasförmigem Zustand in dafür geeigneten und üblichen Vorrichtungen vorgesehen. Eine Herstellung von Flüssigkeiten aus dem Trägermaterial war nicht vorgesehen. Weiterhin ist es nicht bekannt, pflanzliche Stoffe komplett in Filter- bzw. Trägermaterial zu binden.

Aufgabe der Erfindung ist es, ein kostengünstiges Verfahren zur Herstellung einer mit Inhalts- und Wirkstoffen von Pflanzen angereicherten Flüssigkeit unter Verwendung eines Fräger- und Speichermaterials für Pflanzen und deren Wirkstoffe zu entwickeln, das es ermöglicht die Pflanzen und deren Wirkstoffe in zerkleinerter Form zu binden, über lange Zeit zu speichern und diese beim Ansatz mit Flüssigkeiten so wieder abzugeben, daß eine Verwendung als medizinisches, kosmetisches, therapeutisches Mittel im Sport und Freizeitbereich, aber auch die Verwendung als Reinigungsmittel in Haushalt und Gewerbe möglich ist.

Bei der Verwendung als Glasreinigungsmittel soll gleichzeitig ein Antiblendeffekt bei Autoscheiben während Nachtfahrten erreicht werden. Bei der Verwendung als medizinisches bzw. therapeutisches Mittel soll der Wirkungsgrad der Mittel besser als bei auf herkömmlichen Wege hergestellten Mitteln sein, da es gelingt,
1. den in der Natur vorkommenden Wirkstoffmix zu konservieren,
2. die Wirkstoffe mit hohem prozentualem Anteil wieder an die Flüssigkeit abzugeben, wobei das Trägermaterial gleichzeitig sowohl als Konservierungsmittel aber auch als. Katalysator zur Erhöhung der Wirksamkeit der Flüssigkeit wirkt .

Erfindungsgemäß wird die Aufgabe dadurch gelöst, daß die Pflanzen mit Schneidwerkzeugen so zerkleinert werden, daß eine spinatförmige Masse entsteht. Je nach Pflanzenart wird diese Masse mit einer bestimmten Menge von Gummigranulat in reiner Form bzw. mit einem Gemisch aus Gummigranulat und anhaftenden textilen Bestandteilen, wie es nach der DE-A- 19648551 beschrieben ist, solange gemischt, bis man sie formen kann. Dann wird die entstandene Masse maschinell oder an der Luft getrocknet. Das dabei entstandene Trägermaterial ist in der Lage bis zu 97 Prozent aller Inhaltsstoffe der Pflanzen zu speichern, wobei leicht flüchtige Inhaltstoffe zwischen 11 und 24 Prozent, die übrigen zwischen 65 und 97 Prozent nachgewiesen werden können.

Für den bestimmungsgemäßen Einsatz setzt man das mit Pflanzen angereicherte Trägermaterial mit Flüssigkeit in einem bestimmten Mischungsverhältnis bei definierter Temperatur und Zeiteinheit an, die je nach Verwendungszweck unterschiedlich sind, und zieht die Flüssigkeit ab.

Die so entstandene Lösung ist lange Zeit haltbar und wird je nach verwendetem Pflanzenmaterial unterschiedlich in der Medizin, Kosmetik, als therapeutisches Mittel in Sport und Freizeit oder als Reinigungsmittel für Haushalt, Industrie und Gewerbe eingesetzt.

### Ausführungsbeispiele

1. Brennnesselpflanzen werden mit Schneidwerkzeugen solange zerkleinert bis ein spinatförmiger Brei entsteht. Dieser wird mit Gummigranulat in reiner Form oder mit textilen Bestandteilen gemischtem Gummigranulat (PA AZ 196 48 551.7 vom 12.06.97) solange vermischt, bis eine formbare Masse entsteht. Die entstandene Masse wird in zweckmäßige Formen, beispielsweise Tablettenform mit ca. 3 cm Durchmesser, gebracht und getrocknet.
   Die Tabletten werden vor dem bestimmungsgemäßen Einsatz mit Flüssigkeit, vorzugsweise Wasser, angesetzt und über einen definierten Zeitraum bei einer bestimmten Temperatur stehen gelassen.
   Danach wird die entstandene Flüssigkeit abgezogen und abgefüllt.
   1.1. Diese Flüssigkeit wird als Glasreiniger für Autoscheiben, Autoaußen- und Innenspiegel sowie Autoscheinwerfer verwendet. Die Flüssigkeit wird dazu auf die vorgereinigten Autoscheiben(außen und innen), die Spiegel und die Scheinwerfergläser gesprüht und trockenpoliert. Diese Oberflächenbehandlung bewirkt, daß hochglänzende Scheiben mit besserer Tiefenschärfe entstehen. Die Blendwirkung entgegenkommender Fahrzeuge bei Nachtfahrten wird erheblich verringert. Diese Wirkung ist selbst bei leichter Verschmutzung der Scheiben spürbar. Nach normaler Reinigung ist der Effekt wie bei der Erstbehandlung wieder vorhanden. Die Behandlung der Scheiben und Spiegel führt zu einer deutlichen Verringerung der Blendwirkung entgegenkommender oder zu dicht auffahrender Fahrzeuge.
      Die Behandlung der Scheinwerfergläser ermöglicht eine höhere Lichtausbeute.
   1.2. Die Flüssigkeit hergestellt nach Beispiel 1. wird auf Brillengläser, Lupen oder Linsen aufgetragen. Die Gläser werden danach trockenpoliert. Es entstehen saubere und glänzende Oberflächen. Der Antiblendeffekt bei Nacht tritt ebenfalls ein.
   1.3. Die gleiche Flüssigkeit wird auf Fensterscheiben gesprüht und anschließend ebenfalls trocken poliert. Neben den bereits beschriebenen Eigenschaften sind die Scheiben schmutzabweisend, Verschmutzungen lassen sich leichter wieder entfernen. Das Fensterputzen kann längere Zeit lediglich mit klarem Wasser geschehen, ehe man wieder mit Flüssigkeit nach Beispiel 1 grundiert.
      Gleiche positive Wirkungen der Flüssigkeit beim Einsatz als Reinigungs- und Konservierungsmittel wurden erreicht bei der Behandlung von Fliesen, verchromten Teilen wie Wasserhähnen, Handtuchhalter und Wäschetrocknern usw.
   1.4. Flüssigkeit nach Beispiel 1 wurde als Erfrischungs- und Reinigungswasser verwendet, indem man es durch Einreibung auf die Haut oder durch Einsprühen aufbrachte. Die Wirkungen sind verbessertes Wohlbefinden, Kühlung und porentiefe Reinigung sowie anschließende Ausbildung einer Schutzschicht.
   1.5: Flüssigkeit nach Beispiel 1 eignete sich sehr gut beim Einsatz im Sport- und Freizeitbereich. Es hilft bei Muskelverhärtungen, Prellungen, Muskelkater, Zerrungen usw. Verwendet wird es z.B. als Sprühmittel, Einreibung oder Massagemittel oder als Badezusatz bzw. Aufgußmittel in der Sauna.
   1.6. Flüssigkeit nach Beispiel 1 eignete sich als Haarwuchs-und Haarpflegemittel bei bekannter Anwendung, wie Waschen oder Einreiben bzw. Einsprühen.
   1.7. Flüssigkeit nach Beispiel 1 wurde erfolgreich im Rahmen einer Sauerstofftherapie eingesetzt, indem es als feiner Nebel dem Sauerstoff an der Sprühdüse zugegeben wurde.
   1.8. Flüssigkeit nach Beispiel 1 wird auf Kunststoff-Flächen aufgetragen und anschließend poliert. Dabei werden Verschmutzungen, Insekten und deren Ausscheidungen oder Nikotinablagerungen schonend beseitigt. Gleiche gute Ergebnisse wurden beim Auftragen auf Ceranfelder erreicht.
   1.9. Flüssigkeit, hergestellt nach Beispiel 1 wurde mit weiteren pflanzlichen Stoffen und oder Algen in flüssiger oder trockener Form gemischt. Aus dem Gemisch lassen sich therapeutische und kosmetische Produkte herstellen. Das Gemisch ist auch geeignet zur Zugabe als Wirkstoff für Creme, Einreibungs- oder Sprühmittel wie sie in der Kosmetik bzw. Heilbehandlung Anwendung finden.
2. Heilpflanzen oder Pflanzengemische werden mit Schneidwerkzeugen solange zerkleinert bis spinatförmiger Brei entsteht. Dieser wird mit Gummigranulat in reiner Form oder mit textilen Bestandteilen gemischtem Gummigranulat (PA AZ 196 48 551.7 vom 12.06.97) solange vermischt bis eine formbare Masse entsteht. Die entstandene Masse wird in zweckmäßige Formen, beispielsweise Tablettenform mit ca. 3 cm Durchmesser, gebracht und getrocknet.
   Die Tabletten werden vor dem bestimmungsgemäßen Einsatz mit Flüssigkeit, vorzugsweise Wasser, angesetzt und über einen definierten Zeitraum bei einer bestimmten Temperatur stehen lassen. Danach wird die entstandene Flüssigkeit abgezogen und abgefüllt.
   Die Verwendung erfolgt dann ebenfalls durch Einreibung, Aufsprühen, Baden usw.
3. Algen in trockener oder flüssiger Form werden mit zerkleinerten Brennnesselpflanzen oder Flüssigkeit nach Beispiel 1 und Gummigranulat in reiner Form oder mit textilen Bestandteilen gemischten Granulat (PA AZ 196 48 551.1) solange gemischt, bis ein formbarer Brei entsteht. Die entstandene Masse, entsprechend geformt und getrocknet, kann die Bestandteile des Gemisches über lange Zeit speichern. Dem bestimmungsgemäßen Einsatz führt man die getrocknete Masse wie in den Beispielen 1 und 2 beschrieben zu.

## Patentansprüche

1. Verfahren zur Herstellung einer mit Inhalts- und Wirkstoffen von Pflanzen angereicherten Flüssigkeit unter Verwendung eines Träger- und Speichermaterials für Pflanzen und deren Wirkstoffe,
dadurch **gekennzeichnet,** dass Pflanzen, Pflanzengemische oder Gemische aus Pflanzen und Algen mit Schneidwerkzeugen derart zerkleinert werden, dass eine spinatförmige Masse entsteht, diese Masse je nach Pflanzenart mit einer bestimmten Menge von Gummigranulat in reiner Form, Gummigranulat mit anhaftenden textilen Bestandteilen bzw. mit einem Gemisch aus Gummigranulat und textilen Bestandteilen solange gemischt wird, bis man sie formen kann, die so entstandene Masse in geeignete Form bringt, maschinell oder an der Luft trocknet, dadurch die gesamte Pflanzenmasse und ihre Inhaltsstoffe bindet, über lange Zeit speichert, ohne dass sie verdirbt, und dass das mit Pflanzen und deren Wirkstoffen angereicherte Träger- und Speichermaterial mit Flüssigkeit je nach späterem Einsatzzweck mit einer bestimmten Temperatur und über eine bestimmte Zeit angesetzt und die dann so mit Wirkstoffen angereicherte Flüssigkeit abgezogen und abgefüllt wird.

2. Mit Inhalts- und Wirkstoffen von Pflanzen angereicherte Flüssigkeit,
dadurch **gekennzeichnet,** dass die Flüssigkeit entsprechend des Verfahrens gemäß Anspruch 1 hergestellt wurde.

3. Verfahren zur Herstellung einer mit Inhalts- und Wirkstoffen von Pflanzen angereicherten Flüssigkeit unter Verwendung eines Träger- und Speichermaterials für Pflanzen und deren Wirkstoffe,
dadurch **gekennzeichnet,** dass Flüssigkeit, hergestellt gemäß Anspruch 1 bzw. Flüssigkeit gemäß Anspruch 2 mit weiteren Pflanzen, Pflanzengemischen oder Gemischen aus Pflanzen und Algen sowie mit einer bestimmten Menge Gummigranulat in reiner Form, Gummigranulat mit anhaftenden textilen Bestandteilen bzw. einem Gemisch aus Gummigranulat und textilen Bestandteilen solange gemischt wird, bis man sie formen kann, die so entstandene Masse in geeignete Form bringt, maschinell oder an der Luft trocknet, dadurch die gesamte Masse und ihre Inhaltsstoffe bindet, über lange Zeit speichert, ohne dass sie verdirbt, und danach die so entstandene Masse mit Flüssigkeit je nach späterem Einsatzzweck mit einer bestimmten Temperatur über eine bestimmte Zeit ansetzt und die dann mit Wirkstoffen angereicherte Flüssigkeit abzieht und abfüllt.

4. Mit Inhalts- und Wirkstoffen von Pflanzen angereicherte Flüssigkeit,
dadurch **gekennzeichnet,** dass die Flüssigkeit entsprechend des Verfahrens gemäß Anspruch 3 hergestellt wurde.

5. Verwendung von Flüssigkeit, erhältlich nach einem der Verfahren gemäß der Ansprüche 1 und 3 als Reinigungsmittel.

6. Verwendung gemäß Anspruch 5,
dadurch **gekennzeichnet,** dass das Reinigungsmittel zur Reinigung, Versiegelung sowie als Antiblendmittel für Autoscheiben, Spiegel oder Brillen dient.

7. Verwendung von Flüssigkeit, hergestellt nach einem der Verfahren gemäß der Ansprüche 1 oder 3 zur Herstellung von kosmetischen, therapeutischen und medizinischen Mitteln.

## Claims

1. Method for producing a liquid enriched with ingredients and active agents from plants using a carrier and storage material for plants and their active agents, **characterised** in that plants, mixtures of plants or mixtures of plants and algae are comminuted by means of cutting tools in such a way that a spinach-like mass is produced, that, depending an the plant species, this mass is mixed with a defined quantity of rubber granules either in pure form or rubber granules with adhering textile components or with a mixture of rubber granules and textile components, until the mass is mouldable, that the mass so produced is shaped in a suitable form, mechanically dried or air-dried, so as to bind the entire plant mass and its ingredients, that it is stored over a long period of time without deterioration, and that the carrier and storage material enriched with plants and their active ingredients is prepared with liquid depending an the future application at a defined temperature and for a defined period of time, and that the liquid thus enriched with active ingredients is then drawn off and bottled.

2. Liquid enriched with ingredients and active agents of plants, **characterised in that** the liquid was produced according to the method of Claim 1.

3. Method for producing a liquid enriched with ingredients and active agents of plants using a carrier and storage material for plants and their active agents, **characterised** in that liquid produced in accordance with Claim 1 or liquid in accordance with Claim 2 is mixed with other plants, plant mixtures or mixtures of plants and algae as well as with a defined quantity of rubber granules either in pure form or rubber granules with adhering textile components or a mixture of rubber granules and textile components until it is mouldable, that the mass so produced is shaped in a suitable form, mechanically dried or air-dried, so as to bind the entire plant mass and its ingredients, that it is stored over a long period of time without deterioration, whereupon, depending an the future application, the resulting mass is prepared with a liquid, at a defined temperature for a defined period of time, and that the liquid enriched with active ingredients is then drawn off and bottled.

4. Liquid enriched with ingredients and active agents of plants, **characterised** in that the liquid was produced according to the method of Claim 3.

5. Application of liquid, produced according to the methods of Claims 1 and 3 as a detergent.

6. Application according to claim 5, **characterised** in that the detergent is used for cleaning, sealing as well as anti-glaring means for car windows, mirrors or glasses.

7. Application of liquid, produced according to one of the methods of claims 1 or 3 for producing of cosmetic, therapeutic and medicinal products.

## Revendications

1. Procédé de préparation d'un liquide contenant des composés et agents actifs végétaux en utilisant un matériau support et de stockage de plantes et de leurs agents actifs caractérisés en ce que des plantes, des mélanges de plantes ou des mélanges de plantes et d'algues sont broyés jusqu'à obtention d'un broyat qui est ensuite mélangé en fonction des plantes utilisées avec une certaine quantité de granules de gomme pure ou avec des granules de gomme contenant des résidus de textiles ou avec des granules de gomme pure et des résidus de textiles jusqu'à ce que l'on obtienne une pâte que l'on peut modeler à la main et qui est ensuite mise en moules, séchée par une machine ou à l'air libre, fixant ainsi toutes les plantes et leurs composés et les gardant pendant longtemps intacts, que le matériau support et de stockage contenant les plantes et leurs agents actifs et placé dans un liquide en fonction de son utilisation ultérieure à une certaine température et pendant un certain temps et que ce liquide ainsi enrichi d'agents actifs est ensuite prélevé et mis en bouteille.

2. Liquide contenant des composés et des agents actifs végétaux caractérisé en ce que le liquide a été préparé selon le procédé de la revendication 1.

3. Procédé de préparation d'un liquide contenant des composés et agents actifs végétaux en utilisant un matériau support et de stockage de plantes et de leurs agents actifs caractérisé en ce que le liquide, soit préparé selon la revendication 1, soit préparé selon la revendication 2, est mélangé avec d'autres plantes, avec des mélanges de plantes ou avec des mélanges de plantes et d'algues et avec une certaine quantité de granules de gomme pure ou avec des granules de gomme contenant des résidus de textiles ou avec des granules de gomme pure et des résidus de textiles jusqu'à ce que l'on obtienne une pâte que l'on peut modeler à la main et qui est ensuite mise en moules, séchée par une machine ou à l'air libre, fixant ainsi toutes les plantes et leurs composés et les gardant pendant longtemps intacts, que le matériau support et de stockage contenant les plantes et leurs agents actifs et placé dans un liquide en fonction de son utilisation ultérieure à une certaine température et pendant un certain temps et que ce liquide ainsi enrichi d'agents actifs est ensuite prélevé et mis en bouteille.

4. Liquide contenant de composés et agents actifs végétaux caractérisé en ce que le liquide a été préparé selon le procédé de la revendication 3.

5. Utilisation comme nettoyant d'un liquide obtenu selon le procédé des revendications 1 et 3.

6. Utilisation d'un liquide selon revendication 5 caractérisée en ce que le nettoyant sert à nettoyer, à vitrifier et à créer un effet anti-éblouissement pour pare-brises, rétroviseurs et lunettes.

7. Utilisation d'un liquide préparé selon l'un des procédés des revendications 1 ou 3 pour la fabrication de substances cosmétiques et thérapeutiques.
